# EUROPEAN PATENT APPLICATION

(11) **EP 4 660 631 A1**
(43) Date of publication of application: **10.12.2025**
(21) Application number: 23919944.1
(22) Date of filing: 12.12.2023
(51) Int. Cl.: G01N 35/00, G01N 35/04

(54) **STORAGE DEVICE, AUTOMATIC ANALYSIS DEVICE, AND STORAGE METHOD**

(30) Priority: 01.02.2023 JP 2023014250
(71) Applicant: HITACHI HIGH-TECH CORPORATION, Tokyo 105-6409 (JP)
(72) Inventor: MURAMATSU, Yoshiki, Tokyo 105-6409 (JP); YAMASHITA, Taichiro, Tokyo 105-6409 (JP); OKUSA, Takenori, Tokyo 105-6409 (JP); KUMAGAI, Takahiro, Tokyo 105-6409 (JP)
(74) Representative: MERH-IP Matias Erny Reichl Hoffmann Patentanwälte PartG mbB
(86) International application number: PCT/JP2023/044530
(87) International publication number: WO 2024/161809

(57) **Abstract**

To provide a storage device, an automatic analyzer, and a storage method capable of reducing a size of a storage cabinet itself while increasing the number of storage targets that can be stored in the storage cabinet.

A container case 9 in which a QC sample container 11 as a storage target is accommodated and a circular ring holder 8 having a plurality of insertion holes 10 into each of which the container case 9 can be inserted from above are provided. The circular ring holder 8 includes an elastic tongue portion 35 extending upward along a side surface of the container case 9 and elastically deformed in a direction away from a surface of the container case 9. The container case 9 includes a first protrusion 25 that is engaged with the elastic tongue portion 35 to fix the container case 9 when the container case 9 is inserted into the insertion hole 10.

## Description

### Technical Field

The present invention relates to a storage device and a storage method suitable for an automatic analyzer that analyzes a biological sample such as blood or urine, and more particularly, to a storage device, an automatic analyzer, and a storage method for placing a container in which a standard sample or a quality control sample is accommodated in a case separately and performing storage.

### Background Art

In PTL 1, a refrigerated storage assembly integrated with a clinical analyzer includes an insulated housing, an insulated door assembly including one or more doors, one or more thermoelectric coolers, and a refrigerated base assembly contained within the insulated housing and beneath the insulated door assembly. The refrigerated base assembly includes a metal plate thermally coupled to the one or more thermoelectric coolers, one or more thermal sensors, and a plurality of receptacles arranged in an array, each receptacle configured to receive one of a plurality of fluid tubes. It is disclosed that when the one or more doors are closed, the refrigerated base assembly provides a refrigerated environment configured for multi-day storage of at least one of a control and a calibrator fluid contained within the plurality of fluid tubes.

### Citation List

### Patent Literature

PTL 1: JP6715385B

### Summary of Invention

### Technical Problem

An automatic analyzer is a device that automatically analyzes blood and other biological samples and outputs results, and is an essential device in hospitals and medical test facilities. In such an automatic analyzer, it is required to perform a greater variety of tests in a shorter time.

The automatic analyzer requires quality control using a standard sample and a quality control sample when necessary, such as when the automatic analyzer is started up in each morning or when new reagents are added, which places a burden on the operator. Therefore, there is a demand for an automatic analyzer that can achieve quality control without the operator inputting the standard sample or the quality control sample each time.

Such an automatic analyzer requires a storage cabinet for storing the standard sample and the quality control sample. The standard sample and the quality control sample may be collectively referred to as a QC sample or a QC specimen sample.

There are many types of such QC specimen samples since the QC specimen samples differ for each analysis item. Therefore, it is desirable for the storage cabinet to store, for example, 100 or more samples.

Here, to prevent deterioration of the QC samples, contamination with foreign matter, changes in concentration due to evaporation, and the like, it is desirable to perform storage in a cooled state at the time of storage. One example of such a technique is disclosed in PTL 1.

Here, in the refrigerated system as disclosed in PTL 1, the plurality of receptacles are arranged in the array on the refrigerated metal plate, and one of the fluid tubes (containers) is inserted into each receptacle for cooling. Therefore, there is a problem in that when maintenance such as replacing the receptacle into which the container is inserted becomes necessary for some reason, the replacement work is large-scale because it is not possible to replace just one receptacle and it is necessary to replace the metal plate having the plurality of receptacles as a whole. That is, since the maintenance is performed in a collective unit and a work time is required, there is room for improvement in time and cost.

Therefore, to improve the maintenance performance, it is desirable to provide individual container cases. When the individual container cases are used, mixing between different QC samples can be prevented, and thus, a greater advantage is obtained.

However, the number of container cases is equal to the number of stored samples, and it is desirable to have, for example, 100 container cases.

For example, the plurality of container cases are concentrically disposed close to one another in the cooled storage cabinet in a cylindrical shape, and are attached to a substantially disk-shaped holder serving as a base member for holding the container cases.

To simplify the work of attaching the plurality of container cases one by one to the holder provided in the storage cabinet, it is desirable to use a simple configuration without using a special fastening member such as a screw.

As an example of such a mechanism configuration, a so-called snap-fit in which a resin member is extended in a tongue shape and elastic deformation thereof is used to lock two components to each other using a claw is preferable.

In addition, to reduce the size of the storage cabinet and increase the number of container cases to be stored, it is necessary to dispose the container cases at a high density, and it goes without saying that it is desirable that the adjacent container cases can be disposed close to one another. Therefore, there is a demand for a snap-fit arrangement and shape that is suitable for close disposition of the container cases.

To improve maintainability when, for example, it is necessary to replace the container case in the container storage cabinet for some reason, it is desirable to have a configuration in which only the corresponding container case can be removed, attached, and replaced without disassembling the storage cabinet.

In the configuration disclosed in PTL 1, a configuration in which a so-called snap-fit claw for fitting a right case and a left case, and a receiving portion corresponding to the snap-fit claw are provided is disclosed, but a configuration in which a plurality of cases are disposed close to one another is not disclosed.

An object of the invention is to provide a storage device, an automatic analyzer, and a storage method that reduce a size of a storage cabinet itself while increasing the number of storage targets that can be stored in the storage cabinet.

### Solution to Problem

The invention includes a plurality of means for solving the problem, and one example includes: a case in which an article as a storage target is accommodated; and a holder having a plurality of holes into each of which the case is capable of being inserted from above, in which the holder includes a claw portion extending upward along a side surface of the case and elastically deformed in a direction away from a surface of the case, and the case includes a first engagement portion that is engaged with the claw portion to fix the case when the case is inserted into the hole.

### Advantageous Effects of Invention

According to the invention, it is possible to reduce the size of the storage cabinet itself while increasing the number of storage targets that can be stored in the storage cabinet. Problems, configurations, and effects other than those described above will be clarified by the following description of embodiments.

### Brief Description of Drawings

[FIG. 1] FIG. 1 is a schematic plan view of an automatic analyzer according to a first embodiment.
[FIG. 2] FIG. 2 is a schematic plan view of a container storage device according to the first embodiment, and is a cross-sectional view taken along a line B-B in FIG. 3.
[FIG. 3] FIG. 3 is a cross-sectional view taken along a line A-A in FIG. 2.
[FIG. 4A] FIG. 4A is a plan view of a container case of the container storage device according to the first embodiment.
[FIG. 4B] FIG. 4B is a plan view of a holder of the container storage device according to the first embodiment.
[FIG. 5] FIG. 5 is a perspective view of a configuration of the container case and the holder of the container storage device according to the first embodiment as viewed from a front upper side.
[FIG. 6] FIG. 6 is a perspective view of the configuration of the container case and the holder of the container storage device according to the first embodiment as viewed from a lower rear side.
[FIG. 7] FIG. 7 is a longitudinal cross-sectional view showing a snap fit locking operation of the container case and the holder of the container storage device according to the first embodiment.
[FIG. 8] FIG. 8 is a perspective view of the container case and the holder of the container storage device according to the first embodiment in a state after snap fit locking as viewed from the front upper side.
[FIG. 9] FIG. 9 is a perspective view of the container case and the holder of the container storage device according to the first embodiment in a state after the snap fit locking as viewed from the lower rear side.
[FIG. 10] FIG. 10 is a plan view showing an arrangement of a circular ring holder and the container case of the container storage device according to the first embodiment.
[FIG. 11] FIG. 11 is a perspective view of a configuration of the container case, the holder, and a removal fixture of the container storage device according to the first embodiment as viewed from the front upper side.
[FIG. 12] FIG. 12 is a perspective view of the configuration of the container case, the holder, and the removal fixture of the container storage device according to the first embodiment as viewed from the lower rear side.
[FIG. 13] FIG. 13 is a longitudinal cross-sectional view showing a state in which the removal fixture is being inserted into the container case of the container storage device according to the first embodiment from above.
[FIG. 14] FIG. 14 is a longitudinal cross-sectional view showing a state in which the removal fixture is inserted into the container case of the container storage device according to the first embodiment from above to release the snap fit locking.
[FIG. 15] FIG. 15 is a perspective view showing the state in which the removal fixture is inserted into the container case of the container storage device according to the first embodiment from above to release the snap fit locking as viewed from the front upper side.
[FIG. 16] FIG. 16 is a perspective view showing a state in which the container case of the container storage device according to the first embodiment is removed upward from the holder by the removal fixture as viewed from the front upper side.
[FIG. 17] FIG. 17 is a longitudinal cross-sectional view showing an example in which vertical heights of the holder of the container storage device according to the first embodiment are different.
[FIG. 18] FIG. 18 is a plan view showing an arrangement of a holder and a container case of a container storage device according to a second embodiment.

### Description of Embodiments

Hereinafter, embodiments of a storage device, an automatic analyzer, and a storage method according to the invention will be described with reference to the drawings. In the drawings used in the present description, the same or corresponding components are denoted by the same or similar reference signs, and repeated descriptions of these components may be omitted.

### <First Embodiment>

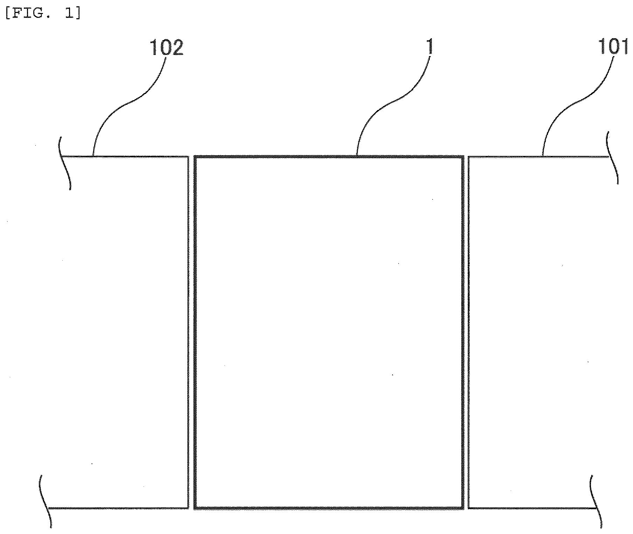

A first embodiment of a storage device, an automatic analyzer, and a storage method according to the invention will be described with reference to FIGS. 1 to 17.

First, an overall configuration of an automatic analyzer including a container storage device 1 will be described with reference to FIG. 1. FIG. 1 is a schematic plan view of the automatic analyzer.

An automatic analyzer 100 according to the invention illustrated in FIG. 1 includes the container storage device 1 that stores a QC sample container 11 used for measurement in an analyzer 101, the analyzer 101 that is a device for measuring a physical property of a sample and needs to ensure analysis accuracy by a QC sample or the like, and a pretreatment device 102 that performs various pretreatments on a sample before analysis on the sample in the analyzer 101 or a post-treatment device that performs various posttreatments on the sample after the analysis on the sample.

One or more of the analyzer 101, the pretreatment device 102, the post-treatment device, and the container storage device 1 that implement the automatic analyzer 100 can be omitted as appropriate, and can be changed as appropriate according to a system configuration.

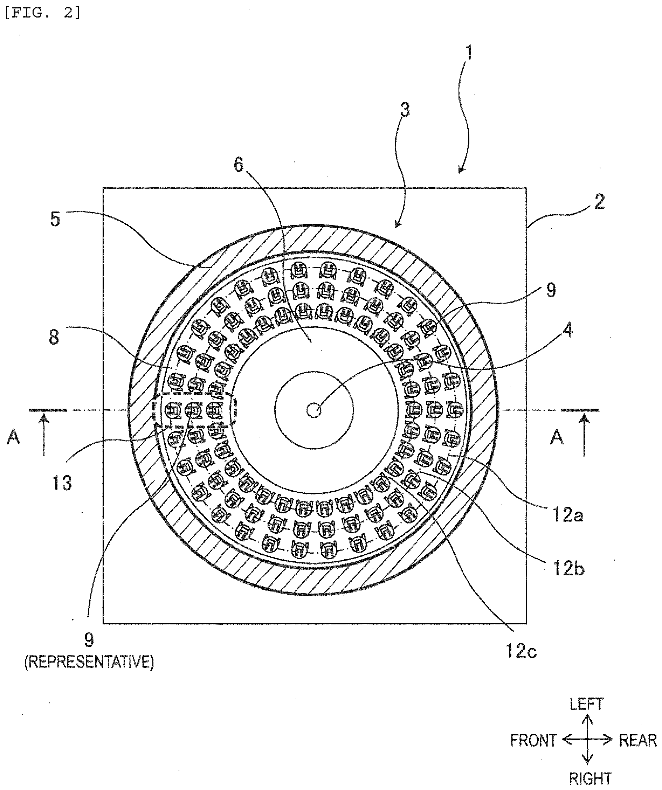

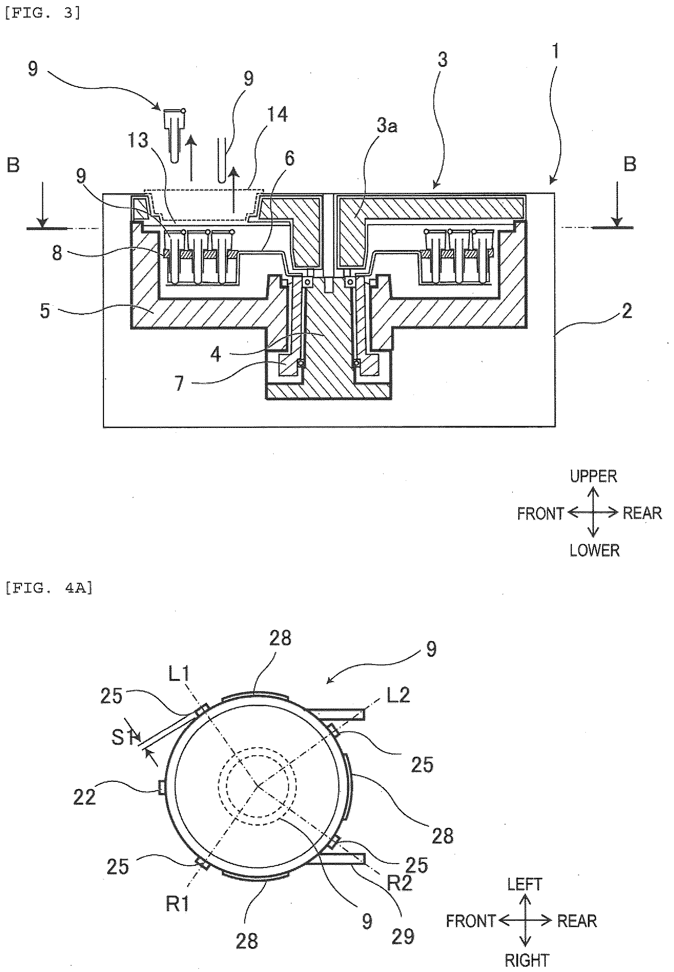

Next, a configuration of the container storage device 1 according to the present embodiment will be described with reference to FIGS. 2 and 3. FIG. 2 is a schematic plan view of the container storage device and is a cross-sectional view taken along a line B-B in FIG. 3. FIG. 3 is a cross-sectional view taken along a line A-A in FIG. 2.

In the present embodiment, an example is illustrated in which a holder is a circular ring holder 8 in which container cases are arranged in three concentric circles, and container cases 9 are concentrically arranged in the circular ring holder 8 with respect to a storage cabinet center shaft 4. However, the holder is not limited to a circular ring shape, and may have any shape of a disk shape or a fan shape, but is not limited to these shapes. The container cases 9 do not need to be concentrically held in the holder, and may be arranged in another manner.

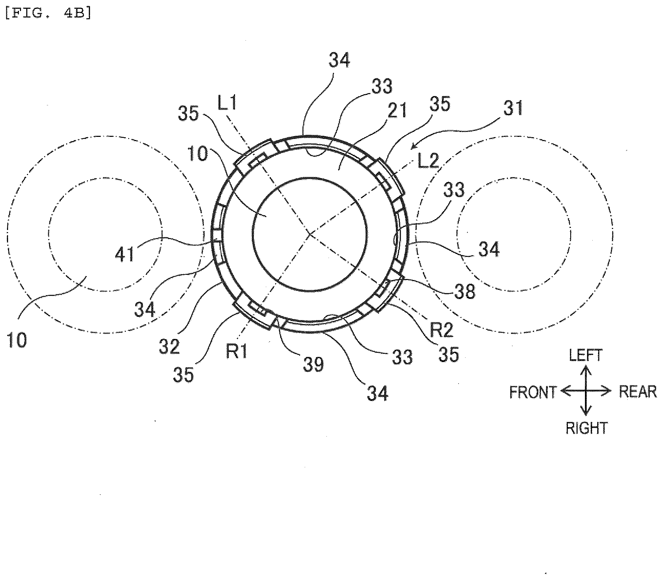

In the following description, up, down, left, right, front, and rear directions are based on up, down, left, right, front, and rear directions illustrated in FIGS. 2 and 3. A front-rear direction is a radial direction of a storage cabinet or a disk along the line A-A, and a left-right direction is a tangential direction of an arc orthogonal to the line A-A. In the description of FIG. 4 and the subsequent drawings, to clearly illustrate the configuration, only a unit configuration corresponding to one in one set of container cases on the central circumference of the three concentric circles and on the line A-A, which is indicated as the "representative" of the circular ring shape holder, is described in detail as a "unit holder", and since the other holders have the same shape as the "unit holder", only the approximate positions thereof are illustrated by dashed-dotted circles.

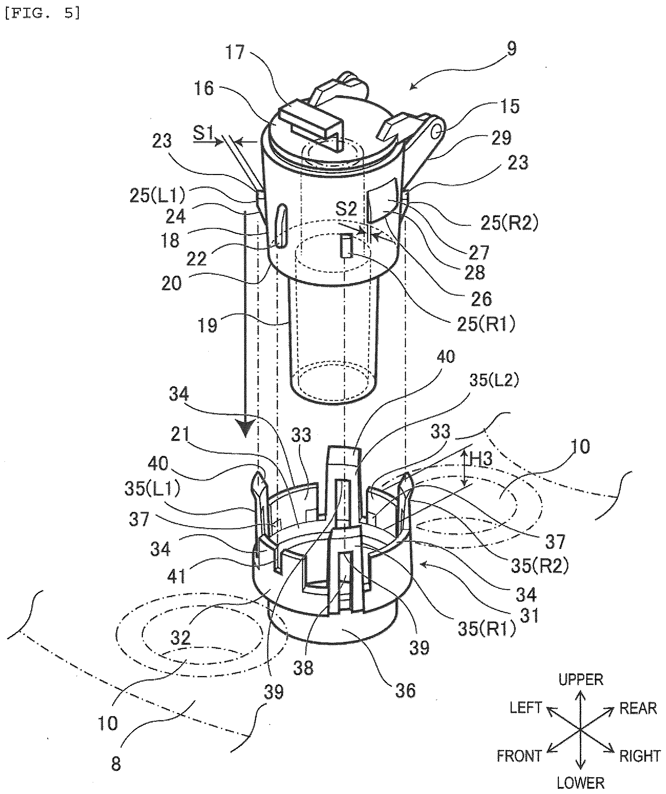

The holder is concentrically arranged using a configuration of the "unit holder" illustrated in FIGS. 4 and 5 as a configuration unit, and is formed in a substantially disk shape as a whole in which the unit holders adjacent to each other in a circumferential direction or a radial direction are formed integrally.

A container may be referred to as a sample container or a QC sample container.

To describe a schematic configuration of the container storage device 1 according to the present embodiment with reference to FIGS. 2 and 3, the container storage device 1 includes a substantially rectangular parallelepiped housing 2, and a substantially cylindrical storage cabinet 3 is provided inside the housing 2. The description of a configuration of a transport unit that transports the QC sample container in and out of the storage cabinet 3 will be omitted.

The storage cabinet 3 is provided in a cylindrical shape about the storage cabinet center shaft 4 oriented in a vertical direction, and to maintain an interior of the storage cabinet 3 at a low temperature, for example, 10 °C or less, the storage cabinet 3 is cooled by a cooling device (not illustrated for convenience of illustration), and a cylindrical surface, an upper surface, and a bottom surface of an outer circumference are covered with a heat insulation material 5 having low thermal conductivity. The upper surface of the storage cabinet 3 is a removable storage cabinet upper surface lid 3a.

A circular or circular-ring-shaped disk 6 pivotally supported to be rotatable about the storage cabinet center shaft 4 is provided inside the storage cabinet 3. The disk 6 is connected via a disk drive shaft 7 to a drive motor (not illustrated), such as a stepping motor, to be rotatable via a timing belt, and can be stopped at a predetermined angular position by an angle detection sensor (not illustrated).

The circular ring holder 8 in a circular ring shape is provided on the disk 6 and is rotatable together with the disk 6. Holes (insertion holes 10) through which a rotary drive shaft or the like passes are provided at a center portion of the circular ring holder 8.

A plurality of the container cases 9 for accommodating the QC sample containers 11 can be attached to the circular ring holder 8, and the insertion holes 10 into which the respective container cases 9 are inserted from above are drilled in number equal to the number of container cases 9 that can be stored. It is desirable that an outer diameter of the circular ring holder 8 is made as large as possible to be substantially equal to an outer diameter of the disk 6, thereby enabling the accommodation of a large number of container cases 9.

In the present embodiment, the container cases 9 are arranged concentrically and radially about the storage cabinet center shaft 4, and the QC sample container 11 is stored in each container case 9. In other words, the container cases 9 are arranged along three concentric circles, that is, along an outer circle 12a, a middle circle 12b, and an inner circle 12c.

An opening 13 is provided in the storage cabinet upper surface lid 3a to take out the QC sample container 11 upward from an inside of the storage cabinet 3. In the present embodiment, the opening 13 has a substantially rectangular shape, and has one side parallel to the line A-A and the other side shorter than the one side and orthogonal to the line A-A. In the present embodiment, a length of the one side is set such that the container cases 9 on the outer circle 12a on the outermost circumference and the inner circle 12c on the innermost circumference, which are arranged in three concentric circles, are exposed in a plan view, and a length of the other side is set such that one container case 9 is exposed in a plan view, making an opening dimension allowing passage of the container case 9 when the container case 9 is moved in an upper-lower direction using a takeout fixture 43 to be described later to be placed on the circular ring holder 8 or taken out of the circular ring holder 8.

The opening 13 is provided with a shutter 14 that is opened and closed by a shutter opening and closing mechanism (not illustrated). To prevent outside air from entering the cooled storage cabinet 3 when the shutter 14 is closed, an airtight sealing material (not illustrated) is provided about the shutter 14, sealing a space between the opening 13 of the storage cabinet 3 and the shutter 14.

When the shutter 14 is fully opened, the shutter 14 is opened to an outside of a projection surface of the opening 13 in the vertical direction, and is disposed not to hinder gripping and takeout of the container case 9 by the removal fixture 43.

In the present embodiment, the QC sample container 11 is stored in the storage cabinet 3 via the circular ring holder 8 in a state of being held in the container case 9 including an opening and closing lid 16 pivotally supported to be openable and closable about a lid support shaft 15 through the opening 13 provided in the upper surface of the storage cabinet 3 and through which the container case 9 and the removal fixture 43 can pass in the upper-lower direction.

When the opening and closing lid 16 is closed, a space between the opening and closing lid 16 and the container case 9 can be kept airtight by an airtight sealing material (not illustrated for convenience of illustration), and the sample in the container case 9 can be prevented from evaporating.

The opening and closing lid 16 is pivotally supported to be openable and closable about the lid support shaft 15 provided on one end of the opening and closing lid 16, and a lid opening claw receiving portion 17 for opening and closing the opening and closing lid 16 by the action of a lid opening and closing mechanism (not illustrated) is provided on the other end of the opening and closing lid 16.

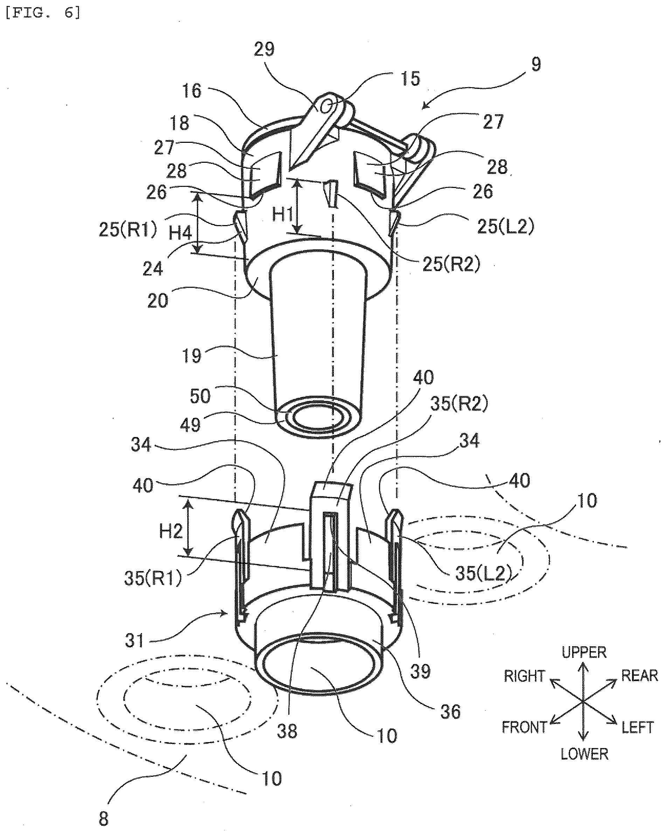

Next, configurations of the container case 9 and the circular ring holder 8 will be described with reference to FIGS. 4A to 6. FIG. 4A is a plan view of the holder, FIG. 4B is a plan view of the holder, FIG. 5 is a perspective view illustrating the configurations of the container case and the holder as viewed from a front upper side, and FIG. 6 is a perspective view illustrating the configurations of the container case and the holder as viewed from a rear lower side.

The container case 9 has a substantially stepped cylindrical shape having an opening on an upper surface thereof, an upper portion is a first cylindrical portion 18 having a large diameter, and a lower portion is a second cylindrical portion 19 concentric with the first cylindrical portion 18 and having a diameter less than that of the first cylindrical portion 18.

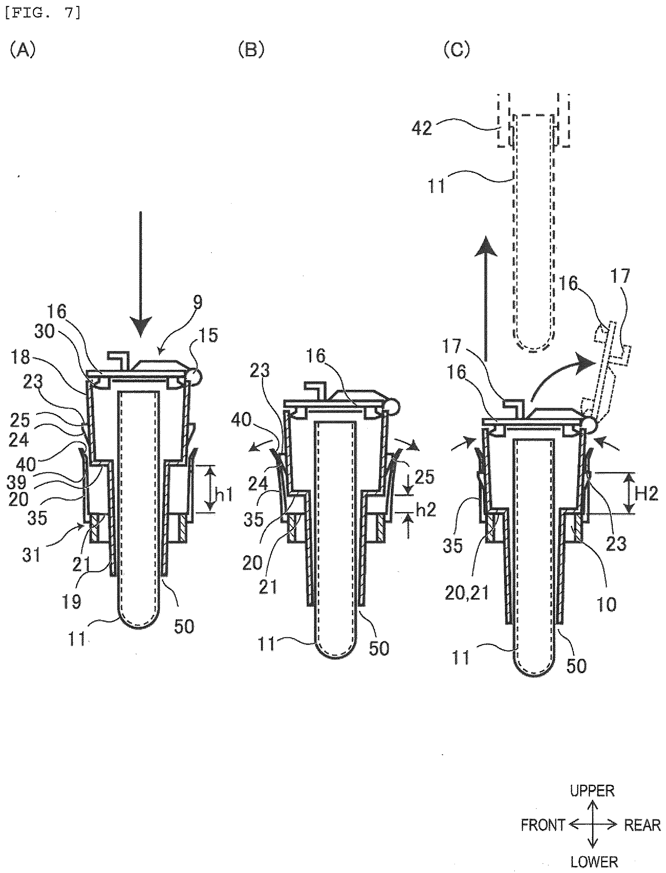

A step portion between an inner circumference of the first cylindrical portion 18 and an inner circumference of the second cylindrical portion 19 is a horizontal placement bottom surface 20 orthogonal to a center shaft of the first cylindrical portion 18 or the second cylindrical portion 19, and can be placed on a case placement surface 21 provided on the circular ring holder 8. A sealing member 50 in a circular ring shape is provided on a case bottom surface 49, which is the lowermost surface of the second cylindrical portion 19, and maintains airtightness with an outer peripheral cylindrical surface of the QC sample container 11 when the QC sample container 11 is inserted as illustrated in FIG. 7.

The container case 9 includes a position determination protrusion 22, first protrusions 25, second protrusions 28, lid support shaft arms 29, and the lid support shaft 15.

The position determination protrusion 22 is a protrusion that is long in the upper-lower direction and protrudes forward from a side surface of the first cylindrical portion 18 along an outer circumference of the first cylindrical portion 18. The first protrusion 25 protrudes from a cylindrical surface of the first cylindrical portion 18 by S1, a horizontal locking surface 23 is formed on an upper surface of the first protrusion 25, and a first sloped surface 24 smoothly connected to an outer circumference of the first cylindrical portion 18 is formed on a lower side. The second protrusion 28 protrudes from the cylindrical surface of the first cylindrical portion 18 by S2, and a second sloped surface 27 smoothly connected to the outer circumference of the first cylindrical portion 18 is formed above a lower surface as a horizontal fixture receiving surface 26.

Among the protrusions described above, the first protrusions 25 engage with elastic tongue portions 35 to fix the container case 9 when it is inserted into the insertion hole 10, and the second protrusions 28 engage with step portions 44 of the removal fixture 43 at side surfaces thereof. Details will be described later.

A sealing portion 30 made of, for example, flexible rubber is provided on a lower surface of the opening and closing lid 16, and is fitted to an inner circumference of the container case 9 to maintain airtightness when the opening and closing lid 16 is closed.

The locking surface 23 of the first protrusion 25 is provided at a position H1 in a height direction from the placement bottom surface 20.

The fixture receiving surface 26 of the second protrusion 28 is provided at a position of H4 in the height direction from the placement bottom surface 20.

As illustrated in FIG. 4A, in a plan view, the first protrusions 25 are provided in four directions of L1, L2, R1, and R2 from a center shaft of the container case 9 connecting the center of the circular ring holder 8 and the center of the container case 9. In the present embodiment, L1 and R1 are disposed symmetrically with respect to a front-rear direction axis, and L2 and R2 are disposed symmetrically with respect to the front-rear direction axis. Therefore, two pairs of the elastic tongue portions 35 paired with the first protrusions 25 are also disposed symmetrically with respect to a line connecting the center of the circular ring holder 8 and the center of the container case 9.

Further, an angle formed by the L1 and the R1 in the plan view is larger than an angle formed by the L2 and the R2. In other words, a distance between the first pair provided farther from the center of the container case 9 than the center of the circular ring holder 8 (a distance between the first protrusions 25 and a distance between the elastic tongue portions 35 provided in the L1 and the R1) is larger than a distance between the second pair provided between the center of the circular ring holder 8 and the center of the container case 9 (a distance between the first protrusions 25 and a distance between the elastic tongue portions 35 provided in the L2 and the R2).

The second protrusions 28 are provided in a total of three directions, including a pair facing right and left and one facing rearward.

The first protrusions 25 provided in the L2 direction and the R2 direction are provided between the lid support shaft arms 29 and the second protrusion 28 facing rearward.

The lid support shaft arms 29 are a pair of arms extending rearward and upward from the first cylindrical portion 18. The lid support shaft 15 is a shaft of the opening and closing lid 16 that opens and closes the opening in the upper surface of the container case 9 near tips of the lid support shaft arms 29.

Next, a shape of the circular ring holder 8 will be described. As described above, the circular ring holder 8 has a configuration in which a plurality of "unit holders 31" each having a basic unit shape capable of holding one container case 9 are arranged side by side and connected to one another or molded integrally, and a disk shape or a circular ring shape capable of concentrically arranging and holding the plurality of container cases 9 as a whole is formed. Therefore, hereinafter, a configuration of the unit holder 31 will be described in detail.

It is desirable that the circular ring holder 8, that is, the unit holder 31 is integrally molded with a resin having elasticity, and may be, for example, a polyacetal (POM) resin.

In FIGS. 4 to 9, for the sake of description, a shape of only one set of the unit holders 31 adjacent to one another on the circular ring holder 8 will be described in detail, and only arrangement positions of the adjacent unit holders 31 are indicated by dash-dotted lines.

The unit holder 31 has a hollow stepped cylindrical shape as a whole, and has a shape that allows the container case 9 to be inserted and removed from above. A step portion provided on an inner circumference side of a first cylinder 32 forming an outer circumference of the unit holder 31 forms a horizontal circular-ring-shaped case placement surface 21. The placement bottom surface 20 of the container case 9 can be placed on the case placement surface 21. Since the case placement surface 21 serves as a placement position reference surface when the container case 9 is locked to the unit holder 31, it is desirable that the case placement surface 21 is processed with high accuracy.

Guide surfaces 33, case guide portions 34, and the elastic tongue portions 35 are provided extending upward from an outer circumference of the case placement surface 21 toward an upper side.

The guide surface 33 is an inner circumferential surface of the case guide portion 34 that has an arc shape and forms a part of a cylinder whose diameter is slightly larger than that of the first cylindrical portion 18 such that an appropriate gap of, for example, about 0.2 mm is provided with the first cylindrical portion 18 of the container case 9 in the plan view from the outer circumference of the case placement surface 21 toward the upper side.

The elastic tongue portion 35 is provided on the circular ring holder 8, extends upward along a side surface of the container case 9, and is a so-called snap-fit that elastically deforms in a direction away from a surface of the container case 9, that is, in a radial direction of the first cylinder 32.

A second cylinder 36 is provided below the case placement surface 21, has a diameter less than that of the first cylinder 32, and is provided with the insertion hole 10 communicating with an inner circumference of the case placement surface 21. A diameter of the insertion hole 10 is larger than that of the second cylindrical portion 19 of the container case 9.

Position determination cylindrical surfaces 37 processed with higher accuracy to make a gap with the container case 9 minute to minimize backlash when the container case 9 is placed may be provided near the case placement surface 21 in the inner circumference of the case guide portion 34. The position determination cylindrical surface 37 may be formed in a discontinuous manner on only a part of the cylindrical surface, rather than on the entire circumference of the cylinder.

The elastic tongue portions 35 are provided to be paired with the first protrusions 25 provided on the container case 9, and are provided in four directions of L1, L2, R1, and R2 in the plan view. By disposing the L1 and the R2, and the L2 and the R1 substantially opposite to each other with respect to a center shaft of the unit holder 31, respectively, the locking when the container case 9 and the unit holder 31 are snap-fit locked is ensured and stable, as to be described later.

The elastic tongue portion 35 has a thin structure having a thickness of, for example, about 1 mm, such that an upper end portion thereof is easily displaced.

Only a portion above a first protrusion receiving surface 39 provided horizontally near an upper end of the elastic tongue portion 35 is connected in the circumferential direction, and a portion below the first protrusion receiving surface 39 is divided by a slit 38 which is an opening.

The first protrusion receiving surface 39 is provided at a height H2 from the case placement surface 21. The H2 is, for example, about 0.2 mm larger than the height H1 from the placement bottom surface 20 of the container case 9 to the locking surface 23 of the first protrusion 25. The elastic tongue portion 35 has a shape inclined upward in a direction away from a cylindrical inner circumference of the unit holder 31 near the upper end of the elastic tongue portion 35, with an inside being a guide slope 40.

A total of four case guide portions 34 each having an arc shape are provided between the adjacent first protrusions 25, that is, between the L1 and the L2, between the L1 and the R1, between the R1 and the R2, and between the L2 and the R2. The case guide portion 34 is not thin, for example having a thickness of about 2 mm, has a horizontal cross section of an arc shape, has a second moment of area in the radial direction of the unit holder 31 in the horizontal cross section sufficiently larger than that of the elastic tongue portion 35, has rigidity that does not easily deform, and is provided from the case placement surface 21 to a height of H3.

A groove portion 41 opened in the upper-lower direction is provided in the case guide portion 34 on a front side of the circular ring holder 8. The groove portion 41 is a protrusion for fixing an orientation of the container case 9 by being engaged with the position determination protrusion 22 when the container case 9 is inserted into the circular ring holder 8 from above, and the position determination protrusion 22 of the container case 9 can be inserted or removed from above. A width of the groove portion 41 in the left-right direction is, for example, about 0.2 mm larger than a width of the position determination protrusion 22 provided on the container case 9 in the left-right direction to provide an appropriate gap.

A dimension of the case guide portion 34 in the upper-lower direction is set to have a dimensional relationship (H4 > H3) with respect to the second protrusion 28, such that the second protrusion 28 and the case guide portion 34 do not interfere with each other when the container case 9 is locked to the unit holder 31.

Next, an operation of inserting the container case 9 into the unit holder 31 from above and locking the container case 9 by snap-fit will be described with reference to FIG. 7. FIG. 7 is a longitudinal cross-sectional view illustrating a snap-fit locking operation.

As illustrated in (A) of FIG. 7, the second cylindrical portion 19 of the container case 9 is inserted into the insertion hole 10 of the unit holder 31 from above, a distance between the placement bottom surface 20 and the case placement surface 21 is h1, and the first protrusion 25 does not act on the elastic tongue portion 35 or the guide slope 40.

As illustrated in (B) of FIG. 7, since the distance between the placement bottom surface 20 and the case placement surface 21 becomes h2 (< h1), when the container case 9 is lowered, the first sloped surface 24, which is a lower surface of the first protrusion 25, acts on the guide slope 40 of the upper end of the elastic tongue portion 35, and the elastic tongue portion 35 is lowered while being elastically deformed in a direction away from the outer circumferential surface of the first cylindrical portion 18.

As illustrated in (C) of FIG. 7, when the container case 9 is further lowered, the case placement surface 21 comes into contact with the placement bottom surface 20. Here, since the locking surface 23 of the first protrusion 25 is at the height H1 from the placement bottom surface 20, the first protrusion receiving surface 39 of the elastic tongue portion 35 is at the height H2 from the placement bottom surface 20, and the H2 is larger than the height H1 by, for example, about 0.2 mm, the first protrusion receiving surface 39 is located above the locking surface 23. Therefore, the elastic deformation of the elastic tongue portion 35 returns to an original shape, and the first protrusion receiving surface 39 is located above the locking surface 23 and acts as a hook, thereby locking the container case 9 to the unit holder 31 while preventing the container case 9 from moving upward.

At the same time, since the position determination protrusion 22 of the container case 9 is inserted into the groove portion 41 of the unit holder 31, angular positioning with respect to the unit holder 31 is performed even when the container case 9 has a cylindrical shape.

Here, when a claw (not illustrated) provided on a lid opening and closing device (not illustrated) is locked to the lid opening claw receiving portion 17 provided on the opening and closing lid 16 and moved upward and rearward to be in a fully open state as illustrated by a broken line, an upper surface of the container case 9 is opened. Next, from this state, the QC sample container 11 held in the container case 9 can be gripped and lifted by using a container gripping device (not illustrated) including a gripper 42 that is an openable and closable gripping claw, and the QC sample container 11 can be taken out from above.

Conversely, the gripper 42 can grip and lower the QC sample container 11, and can insert and set the QC sample container 11 into the empty container case 9.

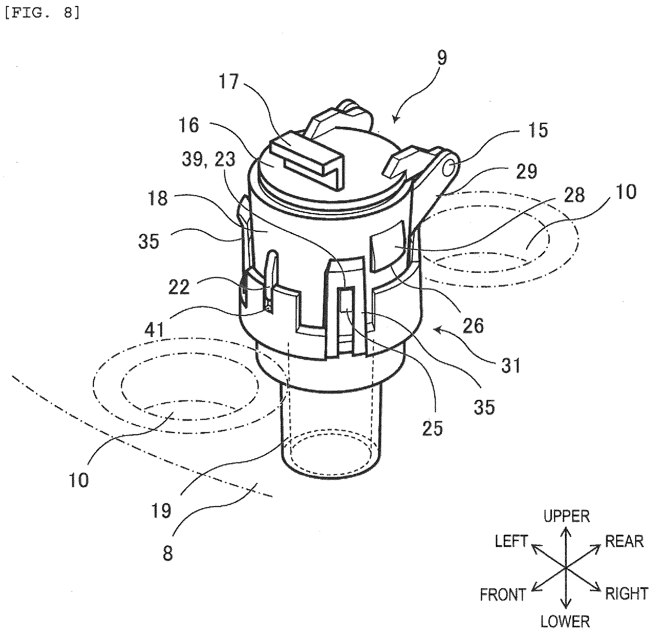

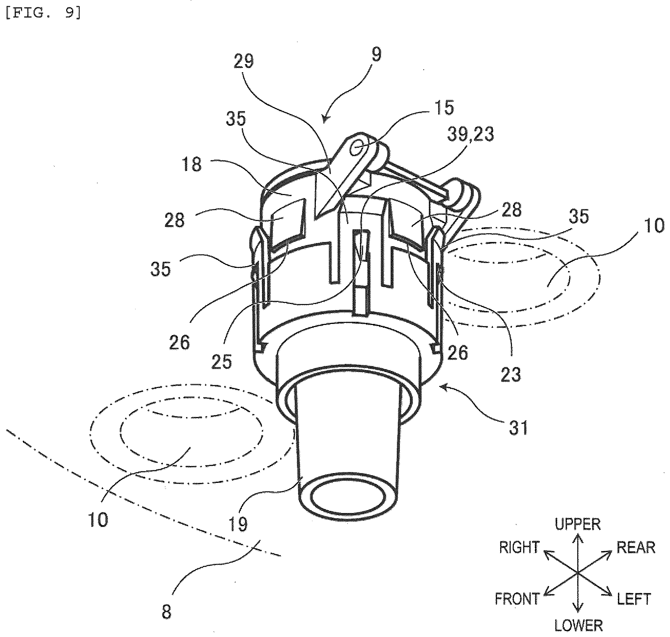

Next, a state in which the container case 9 is locked to the unit holder 31 in the same manner as illustrated in (C) of FIG. 7 will be described with reference to FIGS. 8 and 9. FIG. 8 is a perspective view of a state after snap-fit locking viewed from the front upper side, and FIG. 9 is a perspective view of a state after snapping locking viewed from the rear lower side.

As illustrated in FIGS. 8 and 9, the case placement surface 21 is in contact with the placement bottom surface 20, the elastic tongue portion 35 is locked to the first protrusion 25 at the locking surface 23, and the position determination protrusion 22 is fitted into the groove portion 41 for positioning.

According to the present embodiment, since the container case 9 is locked and positioned by a so-called snap-fit only by inserting the container case 9 into the insertion hole 10 opened in the unit holder 31 from above, it is possible to provide a container storage device in which the attachment of the container case 9 is easy, and particularly when a large number of container cases 9 are to be attached to the circular ring holder 8, they are easily attached in a short time and easily assembled.

Since locking by the elastic tongue portion 35, that is, a so-called snap-fit, is performed at four locations on the circumference of each container case 9, it is necessary to simultaneously release the snap-fit at four locations to remove the container case 9 from the unit holder 31. Therefore, for example, even when foreign matter hits the elastic tongue portion 35 at one location and the locking is temporarily released, the container case 9 is not released from the unit holder 31 or the circular ring holder 8 or the position of the container case 9 is not shifted. Therefore, there is an effect that a highly reliable locking mechanism of the container case 9 can be provided.

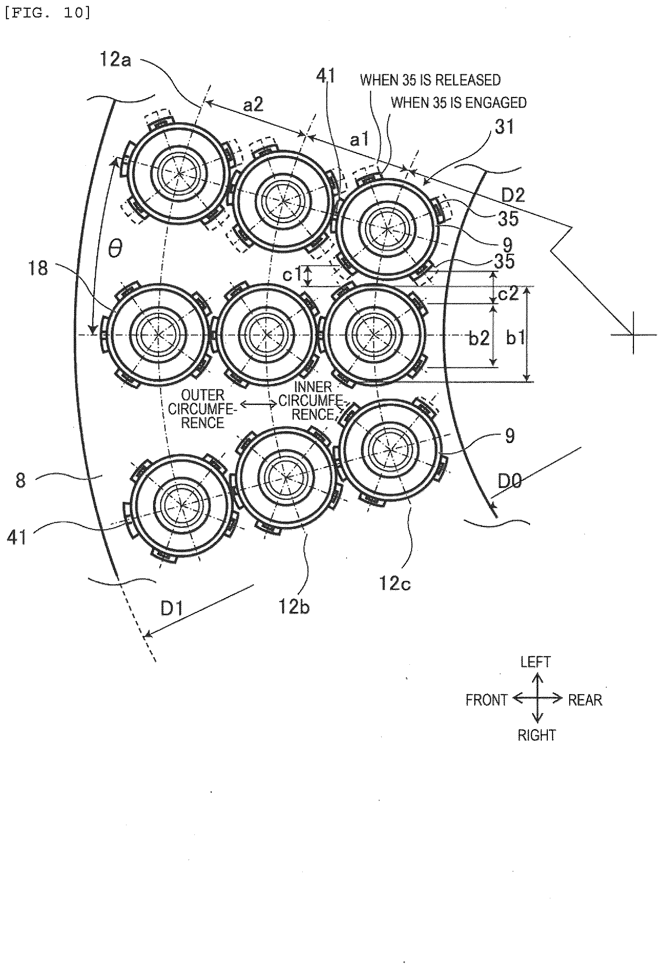

Next, with reference to FIG. 10, a description will be given of a configuration in which a large number of unit holders 31 and container cases 9 are densely provided in the circular ring holder 8 that has a disk shape and has an outermost diameter D1 and an innermost diameter D0 in the storage cabinet 3. FIG. 10 is a plan view illustrating an arrangement of the holder and the container case. In FIG. 10, the opening and closing lid 16 of the container case 9 is omitted.

In the present embodiment, for example, the configuration is implemented in which the unit holders 31 and the container cases 9 are arranged in three concentric circles, and therefore, to arrange the unit holders 31 and the container cases 9 at a high density, it is desirable that adjacent unit holders 31 and container cases 9 on the circumference can be arranged close to one another, and that a radial difference a1 between the inner circle and the middle circle and a radial difference a2 between the middle circle and the outer circle can be minimized to arrange the circles close to one another. When the unit holders 31 having the same shape can be arranged on the inner circle, the middle circle, and the outer circle, the container cases 9 having the same shape can be attached to and detached from the inner circle, the middle circle, and the outer circle, and the container cases 9 can be shared, which is preferable.

First, when the configuration of arrangement close to each other on the circumference is described, when the arrangement is on the inner circumference where a diameter D2 is minimum, it goes without saying that the adjacent unit holders 31 are closest to one another. When a front-rear direction of the unit holder 31 is defined as an orientation of the unit holder 31, since the orientation of the unit holder 31 faces the storage cabinet center shaft 4, the adjacent unit holders 31 are inclined by an angle θ with respect to each other.

At this time, a schematic shape when the elastic tongue portion 35 is elastically deformed as illustrated in (B) of FIG. 7 when the container case 9 is inserted from above and locked is illustrated by a broken line.

As described above, the elastic tongue portion 35 is elastically deformed in a direction away from the outer circumferential surface of the first cylindrical portion 18 of the container case 9. In this way, the orientations (L1, L2, R1, and R2 in FIG. 4) of the elastic tongue portions 35 are set such that the elastic tongue portions 35 do not interfere with the elastic tongue portions 35 provided in the adjacent unit holders 31 even when the elastic tongue portions 35 are elastically deformed. Since it is not necessary to insert or remove two container cases 9 at the same time but one at a time, it is sufficient that the deformed elastic tongue portion 35 does not interfere with the non-deformed elastic tongue portion 35 that locks the adjacent container case 9.

Here, the L1 and the R1 are on an outer circumference side away from the storage cabinet center shaft 4, and the L2 and the R2 are on an inner circumference side close to the storage cabinet center shaft 4. Since a circumferential length on the inner circumference side of a circle is less than a circumferential length on the outer circumference side, it is desirable to arrange the orientation of the L1, the L2, the R1, and the R2 such that a distance b1 between the pair of elastic tongue portions 35 arranged on the L1 and the R1 on the outer circumference side and a distance b2 between the pair of elastic tongue portions 35 arranged on the L2 and the R2 on the inner circumference side satisfy a relationship b1 > b2.

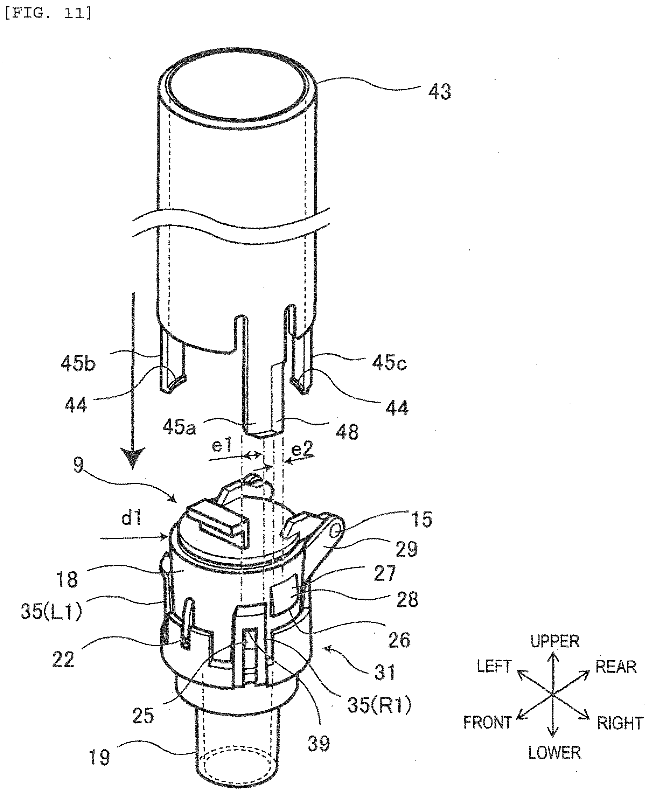

Next, a configuration and an operation of the removal fixture 43 for removing the container case 9 locked by the snap-fit from the unit holder 31 will be described with reference to FIGS. 11 to 16.

As described above, the container case 9 is locked by two pairs of snap-fit that are substantially opposed to each other. Therefore, when the container case 9 is removed due to some trouble, it is desired to move the container case 9 upward while releasing the four snap-fit at the same time.

As illustrated in FIG. 3, in the storage cabinet 3, the container case 9 is located below the storage cabinet upper surface lid 3a in which the heat insulation material 5 having a thickness of, for example, about 40 mm is present from the upper surface. When the shutter 14 is fully opened, only an upper surface of the opening and closing lid 16 is substantially visible through the opening 13.

Although all of the container cases 9 can be accessed from above by disassembling the storage cabinet 3 and removing the storage cabinet upper surface lid 3a, disassembling and reassembling the storage cabinet 3 require extensive work and take time. If such work is performed on an analysis system in operation, the analysis system is stopped for a long time.

Even when the storage cabinet upper surface lid 3a is removed, the operability is not necessarily good because the elastic tongue portions 35 forming the snap-fit are arranged close to one other as illustrated in FIG. 10. In particular, to remove the container case 9 on the inner circumference side, it is not impossible to simultaneously deform the elastic tongue portions 35 at four locations using, for example, a flat-head screwdriver from a narrow gap with the adjacent container cases 9, but this is a difficult task.

Further, if such a task is performed manually, the elastic tongue portions 35 may be deformed too much, causing plastic deformation, which may result in damage to the circular ring holder 8.

Therefore, the present embodiment provides the removal fixture 43 for the container case 9, which can be accessed from above via the opening 13 without removing the storage cabinet upper surface lid 3a, can be inserted between the elastic tongue portion 35 and the side surface of the container case 9 from above, can remove the container case 9 from the circular ring holder 8, and can prevent plastic deformation of the elastic tongue portion 35 as much as possible.

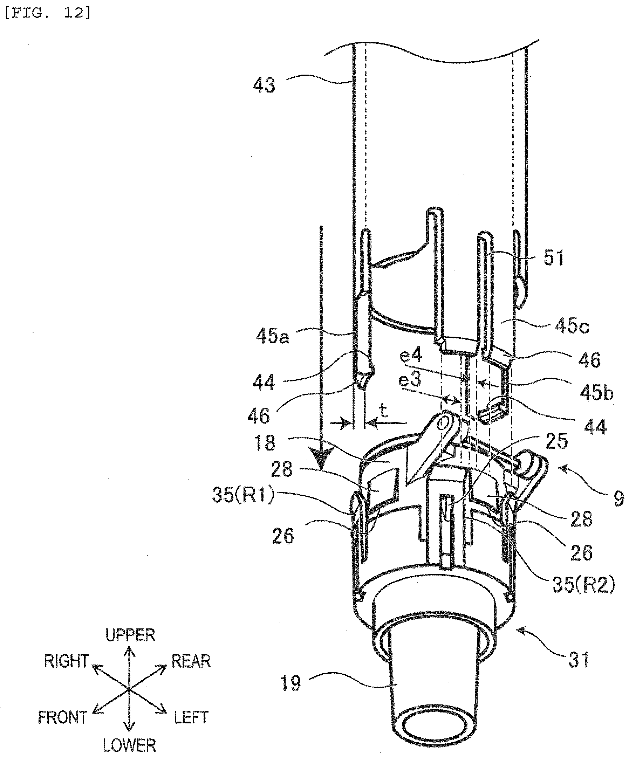

Hereinafter, details of the removal fixture 43 will be described with reference to FIGS. 11 and 12. FIGS. 11 and 12 are perspective views illustrating the configuration of the removal fixture 43 and a positional relationship between the removal fixture 43 and the container case 9 snap-fit locked to the unit holder 31.

As illustrated in FIGS. 11 and 12, the removal fixture 43 has a substantially cylindrical shape, and has an inner diameter slightly larger than a maximum outer diameter of the container case 9.

Second elastic tongue portions 45a, 45b, and 45c at three locations extend downward from a lower end surface of the removal fixture 43, and are provided with the step portions 44 protruding inward at lower end portions, respectively. An outer diameter of the removal fixture 43 is substantially equal to a diameter obtained by adding the outer diameter of the container case 9 and a thickness of the removal fixture 43.

The second elastic tongue portion 45a provided on a right side of the unit holder 31 is provided to act on both the elastic tongue portion 35 provided on the unit holder 31 and the second protrusion 28 provided on the container case 9.

Therefore, a range of a region e1 is inserted between the elastic tongue portion 35 (R1) and an outer circumference surface of the first cylindrical portion 18, and acts to open the elastic tongue portion 35 (R1) and release the locking with the first protrusion 25. A range of a region e2 climbs over the second protrusion 28, and the step portion 44 is locked to the fixture receiving surface 26 of the second protrusion 28 when reaching the lowest point.

A second elastic tongue portion 45 (R1) may be provided with a chamfer 48 for avoiding interference with the adjacent container case 9.

The second elastic tongue portion 45b provided on a left side of the unit holder 31 is symmetrical to the second elastic tongue portion 45a on the right side.

The second elastic tongue portion 45c extending downward is provided on a rear side of the removal fixture 43, and is provided with the step portion 44 protruding inward at the lower end portion.

A range of a region e3 of the second elastic tongue portion 45c is inserted between the elastic tongue portion 35 (R2) and the outer circumference surface of the first cylindrical portion 18, and acts to open the elastic tongue portion 35 (R2) and release the locking with the first protrusion 25. A range of a region e4 climbs over the second protrusion 28, and the step portion 44 is locked to the fixture receiving surface 26 of the second protrusion 28 when reaching the lowest point.

The second elastic tongue portion 45c may be provided with a slit 51 extending in the upper-lower direction at a center, and by providing the slit 51, the second elastic tongue portion 45c is easily bent, making the locking with the second protrusion 28 reliable. The second elastic tongue portion 45c symmetrically has regions having the same action as the region e3 and the region e4 with the slit 51 sandwiched therebetween.

An outer side of a lower end of the second elastic tongue portion 45 is formed as a sloped surface 46 to be easily inserted between the outer circumference surface of the first cylindrical portion 18 and the guide slope 40 at the upper end of the elastic tongue portion 35. A chamfer portion may be provided on an inside of a lower end of the second elastic tongue portion 45 such that the lower end of the second elastic tongue portion 45 smoothly lowers without being caught by an edge of the upper end of the first cylindrical portion 18 of the container case 9.

In the second elastic tongue portion 45, when a thickness from an inner periphery of the step portion 44 to the outer periphery of the removal fixture 43 is set as t, t is larger than the height S1 of the first protrusion 25, that is, a dimensional relationship t > S1 is satisfied.

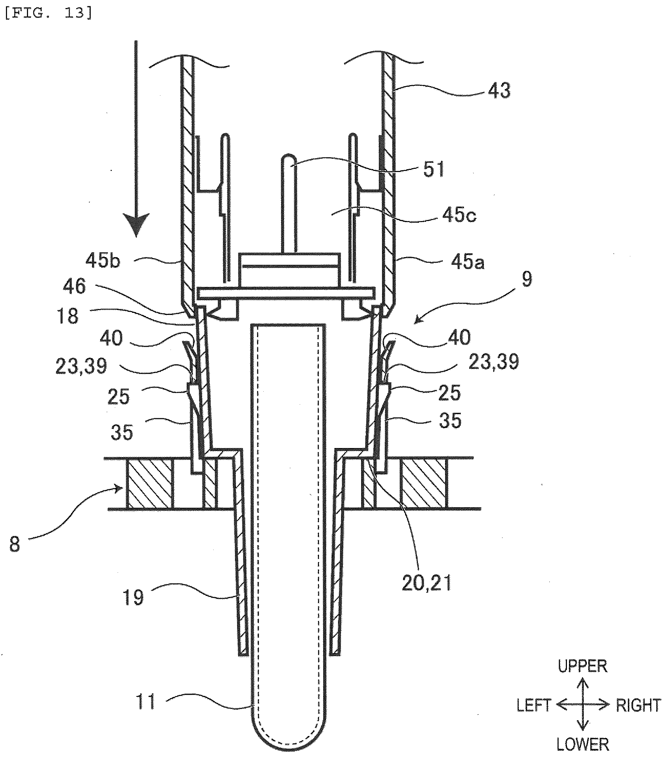

Next, a procedure for removing the container case 9 from the unit holder 31 using the removal fixture 43 will be described with reference to FIGS. 13 to 16.

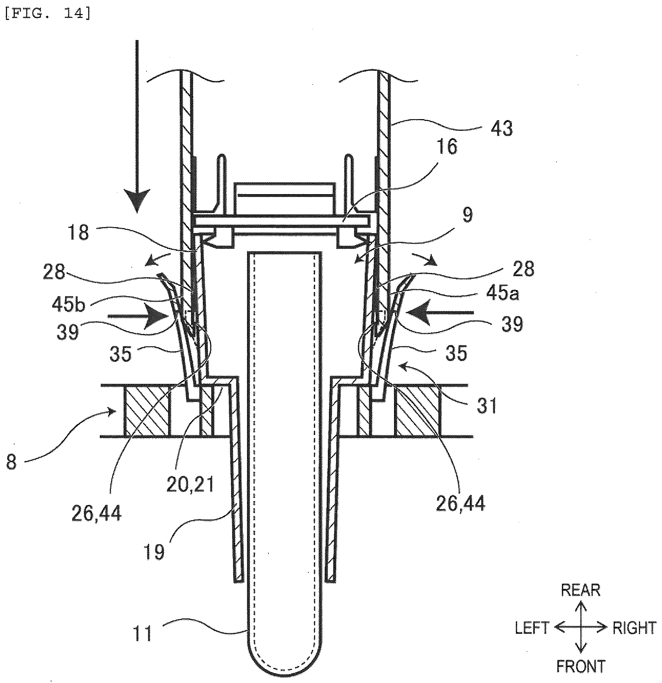

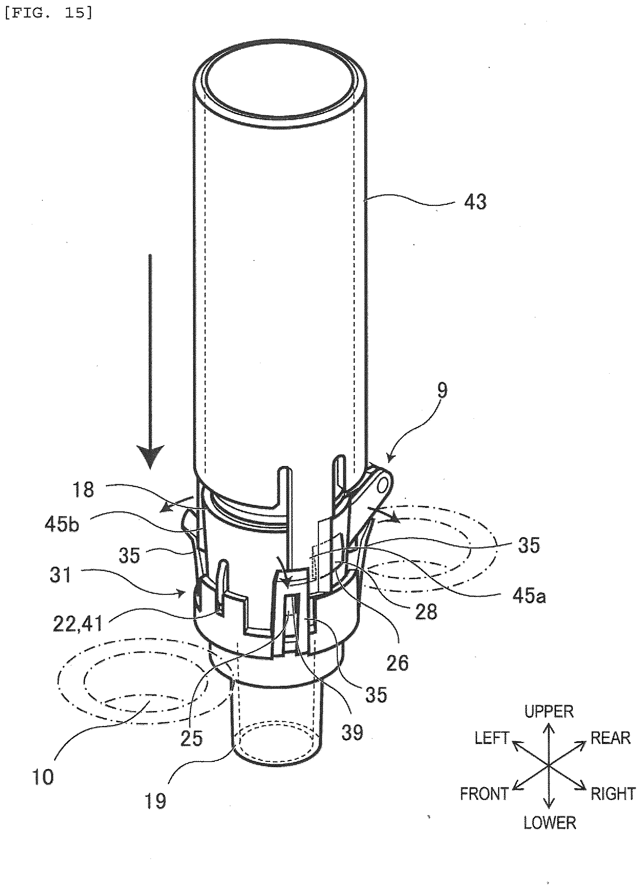

FIG. 13 is a longitudinal cross-sectional view in the left-right direction illustrating a state in which the removal fixture 43 is set immediately above the container case 9 and lowered, and the inner periphery of the lower end of the second elastic tongue portion 45 of the removal fixture 43 is fitted to the outer periphery of the edge of the upper end of the first cylindrical portion 18 of the container case 9 and lowered, but is located above the elastic tongue portion 35 of the unit holder 31. FIGS. 14 and 15 are a longitudinal cross-sectional view and a perspective view illustrating a state in which the removal fixture 43 is further lowered along the outer circumference of the container case 9 from the state of FIG. 13, the second elastic tongue portion 45 is inserted between the elastic tongue portion 35 of the unit holder 31 and the outer circumference of the container case 9, and the step portion 44 of the lower end inner periphery of the removal fixture 43 is locked to the fixture receiving surface 26, which is the lower surface of the second protrusion 28 of the container case 9.

As described above, since the thickness t from the inner periphery of the step portion 44 to the outer periphery of the removal fixture 43 is larger than the height S1 of the first protrusion 25 of the container case 9, in the state of FIGS. 14 and 15, the elastic tongue portion 35 bends to a height equal to or larger than the height of the first protrusion 25, and the snap-fit locking with the first protrusion 25 is released. At this time, since an amount of bend of the elastic tongue portion 35 is limited by the thickness t of the removal fixture 43 and the elastic tongue portion 35 is not bent any more, a maximum value of displacement is constant, and a maximum stress generated at a root of the elastic tongue portion 35 does not exceed a certain value.

That is, it is possible to prevent occurrence of excessive displacement and excessive stress and to prevent the plastic deformation of the elastic tongue portion 35, thereby providing a more reliable container storage device.

The step portion 44 of the lower end inner periphery of the second elastic tongue portion 45 of the removal fixture 43 is locked to the fixture receiving surface 26, which is the lower surface of the second protrusion 28 of the container case 9, and at this time, a reaction force for bending the elastic tongue portion 35 is applied from an outside to an inside of the second elastic tongue portion 45, increasing a pressing force and strengthening the locking between the step portion 44 and the fixture receiving surface 26, thereby ensuring reliable locking, which is preferable.

Further, it is desirable that an outer diameter of the opening and closing lid 16 is slightly less than the outer diameter of the container case 9 such that the inner periphery of the removal fixture 43 and the outer periphery of the opening and closing lid 16 do not interfere with each other.

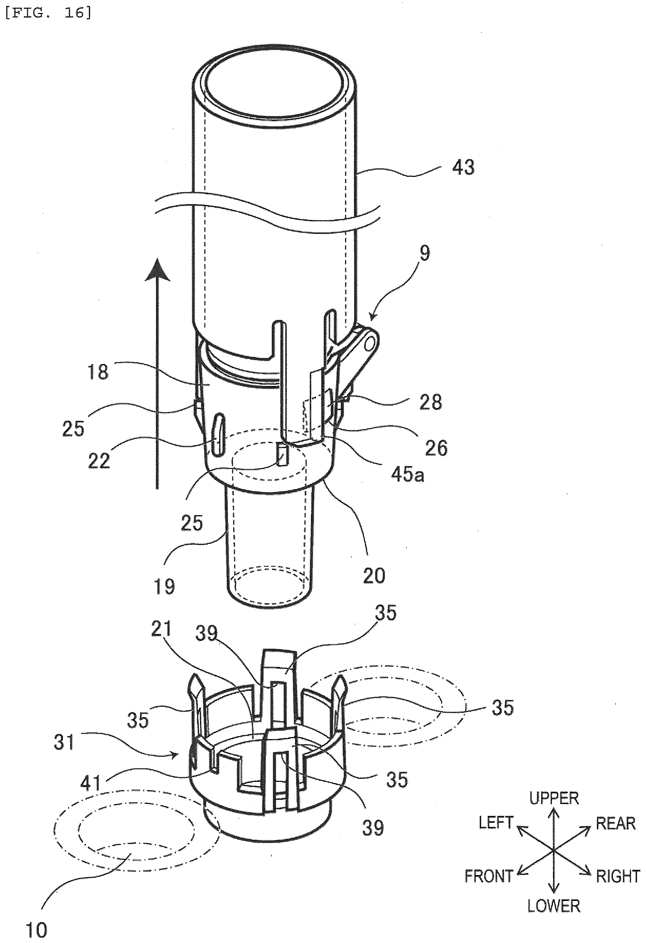

FIG. 16 is a perspective view illustrating a state in which the removal fixture 43 is raised from the state of FIGS. 14 and 15. As described above, since the locking between the elastic tongue portion 35 and the first protrusion 25 is released and the step portion 44 of the second elastic tongue portion 45 and the fixture receiving surface 26 of the second protrusion 28 are locked, the container case 9 can be raised together with the removal fixture 43 and removed from the unit holder 31.

That is, a movement direction of the removal fixture 43 for being engaged with the container case 9 and a movement direction for removing the container case 9 are opposite to each other, the removal fixture 43 is engaged with the container case 9 by being moved downward from above along the outer diameter of the container case 9, and the container case 9 can be removed from the unit holder 31 by moving upward from below after the engagement. There is an effect that the removal operation of the container case 9 is only a simple vertical operation of the removal fixture 43 and is easy, and a complicated operation is not required.

The removal fixture 43 has a cylindrical shape not much different from that of the container case 9, except that a diameter is larger than the outer diameter of the container case 9 by the thickness. Therefore, a task for removing the container case 9 using the removal fixture 43 can be performed through an opening if there is an opening larger than a plan view in the state in which the container case 9 and the removal fixture 43 are engaged.

Here, as described above, by setting the dimension of the opening 13 illustrated in FIGS. 2 and 3 as a dimension that allows passage in the upper-lower direction in the state in which the container case 9 and the removal fixture 43 are locked with each other, it is possible to perform attachment, removal, and the replacement task of the container case 9 from the opening 13.

That is, even when the container case 9 needs to be replaced at the time of maintenance and inspection, since extensive disassembly operation such as removal of the storage cabinet upper surface lid 3a is not required, the operation can be easily performed in a short time, and the stop time of the container storage device 1 or the analyzer 101 connected to the container storage device 1 can be shortened.

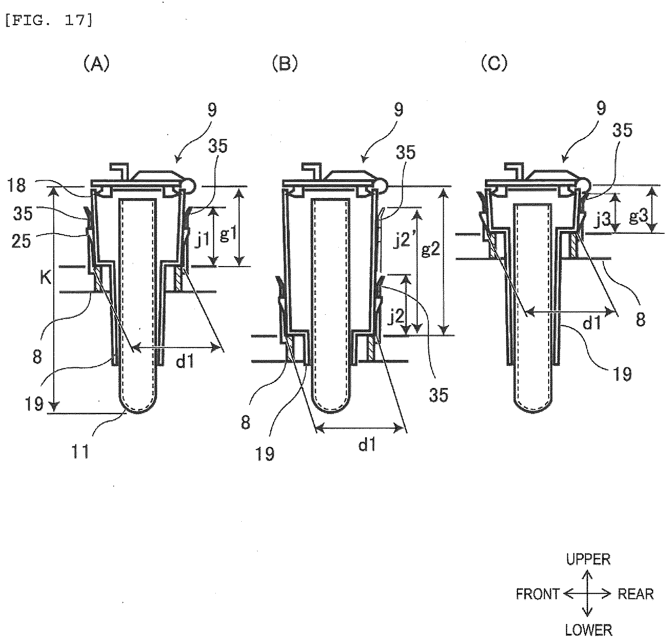

Next, a preferred arrangement relationship between the container case 9 and the unit holder 31 in the upper-lower direction will be described with reference to FIG. 17. FIG. 17 is a longitudinal cross-sectional view illustrating different forms of a height relationship between the holder and the container case.

(A), (B), and (C) of FIG. 17 are longitudinal cross-sectional views illustrating forms in which an overall vertical height K of the container case 9 including the QC sample container 11 is equal, and heights g1, g2, and g3 from an upper surface of the unit holder 31 to the upper surface of the container case 9 are different from vertical heights j1, j2, and j3 of the elastic tongue portion 35 of a snap-fit portion. Since the only difference is the positional relationship between the unit holder 31, the container case 9, and the elastic tongue portion 35 in the upper-lower direction, the same reference numerals are used for portions having the same functions.

(A) of FIG. 17 illustrates a case in which a ratio of a height to the upper surface of the container case 9, that is, the height g1 of the first cylindrical portion 18, to the overall height K, is about 1/3.

For example, when the K = about 80 mm, the g1 = about 28 mm, the j1 = about 15 mm to 20 mm, and the height of the first protrusion 25 is about 1.5 mm, the elastic tongue portion 35, which is a thin resin having a thickness of about 1 mm, can lock the container case 9 without being plastically deformed even when the elastic tongue portion 35 is bent by the height of the first protrusion 25 of about 1.5 mm.

By setting a diameter of the first cylindrical portion 18 as D1 and the D1 ≈ the g1, an error of the upper surface of the container case 9 is about the same as an error of the case placement surface 21 of the unit holder 31, the case placement surface 21 is processed with high accuracy, and therefore the position of the container case 9 can be maintained with high accuracy without increasing the error, which is preferable.

(B) of FIG. 17 illustrates a case in which the unit holder 31 is disposed near the bottom surface of the container case 9. With such an arrangement, the g1 > 50 mm, and the diameter D1 of the first cylindrical portion 18 is about twice as large as the g1. Therefore, the error of the case placement surface 21 of the unit holder 31 is magnified by approximately twice as much on the upper surface of the container case 9.

Here, since the locking by the snap-fit has lower rigidity than, for example, fastening by a screw, it is difficult to further stabilize the positioning in the horizontal plane particularly on the upper surface of the container case 9, and it is desirable to more reliably maintain an interval with the adjacent container case 9. When the length of the elastic tongue portion 35 is set to the j2 ≈ the j1, the elastic tongue portion 35 is recessed from the opening 13 when the container case 9 is inserted from above, and therefore it becomes necessary to improve visibility. Since the length of the second elastic tongue portion 45 of the removal fixture 43 is increased, it is necessary to increase the rigidity of the second elastic tongue portion 45, and further improvement is desired for correctly inserting the lower end of the second elastic tongue portion 45 between the guide slope 40 at the upper end of the elastic tongue portion 35 and the cylindrical surface of the container case 9 when it is inserted downward along the outer circumference of the container case 9.

On the other hand, when the elastic tongue portion 35 is increased in height such that the j2 is set as j2' such that the upper end of the elastic tongue portion 35 approaches the height of the inlet of the container case 9, the rigidity of the elastic tongue portion 35 is reduced, making it difficult to increase the reaction force in the horizontal direction and thereby increase the holding force of the container case 9. When the unit holder 31 or the circular ring holder 8 are resin molded components, a depth of the mold becomes greater depending on the height of the elastic tongue portion 35, and therefore it is necessary to consider to prevent the mold shape from having elongated, weak portions. For example, when the space between the adjacent container cases 9 is increased to increase the thickness of the mold to ensure the strength of the mold, since there is a limit in arranging the container cases 9 close to each other and there is a limit in increasing the density, some ingenuity in arrangement is required.

(C) of FIG. 17 illustrates a case in which the unit holder 31 is arranged in the vicinity of the upper surface of the container case 9, contrary to (B) of FIG. 17. With such an arrangement, since the length j3 of the elastic tongue portion 35 < the j1, it is necessary to take measures to prevent the plastic deformation of the elastic tongue portion 35 at the time of attachment to or detachment from the first protrusion 25 or at the time of removal by the removal fixture 43.

Therefore, as illustrated in (A) of FIG. 17, when the diameter D1 of the first cylindrical portion 18 and the height g1 of the first cylindrical portion 18 are approximately equal as the D1 ≈ the g1 and are about 1/3 of the overall height K, and the height j1 of the elastic tongue portion 35 of the snap-fit is arranged at a position close to the upper surface of the container case 9 at which the visibility from the opening 13 is good, there is an effect that it is possible to implement a configuration suitable for high-density mounting since the container case 9 can be seated well when set on the unit holder 31 or the circular ring holder 8, the positional accuracy of the container case 9 can be easily obtained, the container case 9 can be easily removed and attached, locking by the snap-fit is good, and the adjacent container cases 9 can be arranged close to each other.

Next, effects of the present embodiment will be described.

The container storage device 1 according to the first embodiment of the invention described above includes the container case 9 in which the QC sample container 11 as a storage target is accommodated and the circular ring holder 8 having a plurality of insertion holes 10 into each of which the container case 9 can be inserted from above, the circular ring holder 8 includes the elastic tongue portion 35 extending upward along a side surface of the container case 9 and elastically deformed in a direction away from a surface of the container case 9, and the container case 9 includes the first protrusion 25 that is engaged with the elastic tongue portion 35 to fix the container case 9 when the container case 9 is inserted into the insertion hole 10.

Accordingly, since the container case 9 is locked to the circular ring holder 8 provided inside the storage cabinet by snap-fit, there is an effect that the attachment and the positioning are completed only by pushing the container case 9 from above, a special fastening member such as a screw is not required, and the assembly task can be performed easily and in a short time. Therefore, it is possible to implement a simpler structure for airtightly storing each of containers containing QC samples, in individual container cases 9 and refrigerating and storing the containers, thereby preventing evaporation of the samples and the inclusion of foreign matter, and providing the highly reliable container storage device 1.

In particular, for QC samples, which have an extremely long storage period in the storage cabinet 3 compared to assay reagents and samples and for which even slight evaporation needs to be prevented for quality control, it is desirable to store the QC samples individually in sealed containers rather than simply storing the QC samples in the storage cabinet 3, and this is extremely suitable for storing such QC sample containers 11.

Since the plurality of elastic tongue portions 35 are provided along the side surface of the container case 9 and the plurality of first protrusions 25 are provided to be paired with the plurality of elastic tongue portions 35, the container case 9 can be more firmly and easily fixed to the circular ring holder 8.

The container storage device 1 has the opening 13 on the upper surface side, and by holding a container in which a liquid is accommodated as a storage target, it is possible to provide the container storage device 1 extremely suitable for storing the QC sample container 11 having the characteristics as described above.

Since the container case 9 has a cylindrical shape, when the container cases 9 are concentrically arranged on the circular ring holder 8, the same container case 9 can be placed from the inner circumference to the outer circumference, and therefore there is an effect that the components can be shared.

Since the container case 9 further includes the position determination protrusion 22 protruding from the side surface, and the circular ring holder 8 further includes the groove portion 41 that is engaged with the position determination protrusion 22 to fix the orientation of the container case 9 when the container case 9 is inserted into the circular ring holder 8 from above, positioning is performed as the groove portion 41 functions as a rotation stopper when the container case 9 is locked to the unit holder 31, and there is an effect that the positional accuracy is high.

The holder has any one of a disk shape, a circular ring shape, and a fan shape, the container case 9 is concentrically arranged with respect to the holder, two pairs of the elastic tongue portions 35 and two pairs of the first protrusions 25 are respectively disposed symmetrically with respect to a line connecting a center of the holder and a center of the container case 9, and a distance between a first pair provided farther than the center of the container case 9 with respect to the center of the holder is larger than a distance between a second pair provided between the center of the holder and the center of the container case 9. Accordingly, even when the adjacent unit holders 31 are arranged close to each other, and even when the elastic tongue portions 35 are elastically deformed when the container cases 9 are attached, there is no interference with the adjacent elastic tongue portions 35, and since the unit holders 31 can be arranged close to each other, it is possible to arrange the container cases 9 at high density, which has the effect of increasing the number of container cases 9 to be stored and making the size of the storage cabinet 3 reduced.

The removal fixture 43 capable of being inserted between the elastic tongue portion 35 and the side surface of the container case 9 from above and configured to remove the container case 9 from the circular ring holder 8 is further included, the container case 9 further includes the second protrusion 28 configured to engage with the step portion 44 of the removal fixture 43 in the side surface, and when the removal fixture 43 is inserted between the elastic tongue portion 35 and the side surface and reaches a first predetermined position, the engagement between the elastic tongue portion 35 and the first protrusion 25 is released and the step portion 44 engages with the second protrusion 28, and when the removal fixture 43 is pulled upward, the container case 9 is removed from the insertion hole 10. Accordingly, the container case 9 can be easily removed from and attached to the circular ring holder 8 using the removal fixture 43.

Since a movement direction of the removal fixture 43 for engaging with the container case 9 and a movement direction of the removal fixture 43 for removing the container case 9 are opposite to each other, the container case 9 can be removed from the circular ring holder 8 only by raising the removal fixture 43. Therefore, there is an effect that the container case 9 can be more easily taken out from the opening on the upper surface of the storage cabinet 3 without disassembling the storage cabinet 3, and the replacement of the container case 9 is further facilitated.

The cylindrical storage cabinet 3, the cylindrical disk 6 pivotally supported to be rotatable about the storage cabinet center shaft 4 provided inside the storage cabinet 3, the opening 13 through which the container case 9 and the removal fixture 43 are capable of passing in the upper-lower direction in an upper surface of the storage cabinet 3, and the opening and closing lid 16 capable of opening and closing the opening 13 are further included, the circular ring holder 8 has any one of a disk shape, a circular ring shape, and a fan shape rotating together with the disk 6, the container case 9 is held by the holder concentrically with respect to the storage cabinet center shaft 4, and when the opening and closing lid 16 is opened, the removal fixture 43 is lowered through the opening 13 to engage with the container case 9, the engagement between the container case 9 and the holder is released, and the container case 9 is taken out from the opening 13 by raising the removal fixture 43. Accordingly, since the container case 9 can be replaced through the opening 13, and the container case 9 can be easily replaced in a short time without disassembling the storage cabinet 3 when replacing the container case 9, there is an effect in which the container storage device 1 having good maintainability can be provided.

The container storage device 1 according to the invention is not limited to the embodiment described above.

For example, the circular ring holder 8 has a configuration in which the plurality of unit holders 31 having a basic unit shape are arranged side by side and connected to one another and a disk shape capable of holding the plurality of container cases 9 as a whole is formed. However, the circular ring holder 8 is not limited to such a form, and may be divided into a plurality of fan shapes instead of one disk shape, and the circular ring holder 8 having a disk shape as a whole may be formed by connecting the plurality of fan holders adjacent to each other along an arc.

The circular ring holder 8 may be integrated with or a separated from the disk 6.

The container case 9 has a stepped cylindrical shape, but is not limited to a cylindrical shape, and for example, the container case may have a quadrangular prism shape, and may have a configuration in which the elastic tongue portion and the first protrusion are provided on each side of the quadrangular prism.

### <Second Embodiment>

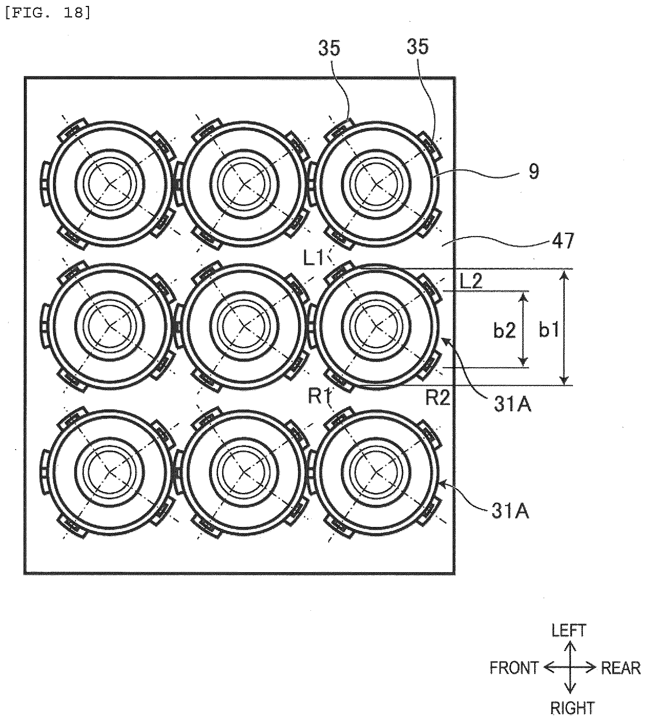

A storage device, an automatic analyzer, and a storage method according to a second embodiment of the invention will be described with reference to FIG. 18. FIG. 18 is a plan view illustrating an arrangement of holders and container cases of the container storage device according to the second embodiment.

FIG. 18 illustrates a form in which the container cases 9 are not arranged concentrically but arranged in a rectangular storage cabinet in a grid shape. In the present embodiment, similarly to the concentric arrangement illustrated in FIG. 10, the elastic tongue portions 35 are provided in the directions of L1, L2, R1, and R2 with respect to a unit holder 31A. The direction of the elastic tongue portion 35 may be different from the position suitable for the concentric arrangement as long as the elastic tongue portion 35 does not interfere with the elastic tongue portion 35 of the adjacent unit holder 31A when the elastic tongue portion 35 is deformed when the container case 9 is attached or removed as described above.

In the present embodiment, since a holder 47 is rectangular and cannot rotate, as illustrated in (C) of FIG. 7, a container gripping device (not illustrated) including the gripper 42 for taking out the QC sample container 11 from the container case 9 may be configured to be freely movable on a plane by, for example, a slide member movable in the front-rear direction and a slide member movable in the left-right direction in a rectangular region in which the container case 9 is disposed.

Other configurations and operations are substantially the same as the configurations and the operations of the storage device, the automatic analyzer, and the storage method according to the first embodiment described above, and details thereof will be omitted.

In the storage device, the automatic analyzer, and the storage method according to the second embodiment of the invention, substantially the same effects as those of the storage device, the automatic analyzer, and the storage method according to the first embodiment described above can be obtained.

### <Others>

The invention is not limited to the embodiments described above and includes various modifications. The embodiments described above have been described in detail to describe the invention in an easy-to-understand manner, and the invention is not necessarily limited to those including all configurations described above.

A part of a configuration according to a certain embodiment can also be replaced with a configuration according to another embodiment, and a configuration according to another embodiment can be added to a configuration according to a certain embodiment. A part of a configuration of each embodiment may be added to, deleted from, or replaced with another configuration.

### Reference Signs List

1: container storage device
2: housing
3: storage cabinet
3a: storage cabinet upper surface lid
4: storage cabinet center shaft
5: heat insulation material
6: disk
7: disk drive shaft
8: circular ring holder
9: container case
10: insertion hole
11: QC sample container (article)
12a: outer circle
12b: middle circle
12c: inner circle
13: opening
14: shutter
15: lid support shaft
16: opening and closing lid
17: lid opening claw receiving portion
18: first cylindrical portion
19: second cylindrical portion
20: placement bottom surface
21: case placement surface
22: position determination protrusion
23: locking surface
24: first sloped surface
25: first protrusion (first engagement portion)
26: fixture receiving surface
27: second sloped surface
28: second protrusion (second engagement portion)
29: lid support shaft arm
30: sealing portion
31, 31A: unit holder
32: first cylinder
33: guide surface
34: case guide portion
35: elastic tongue portion (claw portion)
36: second cylinder
37: position determination cylindrical surface
38: slit
39: first protrusion receiving surface
40: guide slope
41: groove portion
42: gripper
43: removal fixture
44: step portion (third engagement portion)
45, 45a, 45b, 45c: second elastic tongue portion
46: sloped surface
47: holder
48: chamfer
49: case bottom surface
50: sealing member
51: slit
100: automatic analyzer
101: analyzer
102: pretreatment device

## Claims

1. A storage device comprising:
a case in which an article as a storage target is accommodated; and
a holder having a plurality of holes into each of which the case is capable of being inserted from above, wherein
the holder includes a claw portion extending upward along a side surface of the case and elastically deformed in a direction away from a surface of the case, and
the case includes a first engagement portion that is engaged with the claw portion to fix the case when the case is inserted into the hole.

2. The storage device according to claim 1, wherein
a plurality of the claw portions are provided along the side surface of the case, and
a plurality of the first engagement portions are provided to be paired with the plurality of claw portions.

3. The storage device according to claim 2, wherein
the storage device has an opening on an upper surface side, and
holds a container in which a liquid is accommodated as the storage target.

4. The storage device according to claim 3, wherein
the case has a cylindrical shape.

5. The storage device according to claim 4, wherein
the case further includes a protrusion protruding from the side surface thereof, and
the holder further includes a groove that fixes an orientation of the case by engaging with the protrusion when the case is inserted into the holder from above.

6. The storage device according to claim 5, wherein
the holder has any one of a disk shape, a circular ring shape, and a fan shape,
the case is concentrically arranged with respect to the holder,
two pairs of the claw portions and two pairs of the first engagement portions are respectively disposed symmetrically with respect to a line connecting a center of the holder and a center of the case, and
a distance between a first pair of the claw portions or the first engagement portions provided farther than the center of the case with respect to the center of the holder is larger than a distance between a second pair of the claw portions or the first engagement portions provided between the center of the holder and the center of the case.

7. The storage device according to claim 1, further comprising:
a fixture capable of being inserted between the claw portion and the side surface of the case from above and configured to remove the case from the holder, wherein
the case further includes a second engagement portion configured to engage with a third engagement portion of the fixture in the side surface, and
when the fixture is inserted between the claw portion and the side surface and reaches a first predetermined position, the engagement between the claw portion and the first engagement portion is released and the third engagement portion engages with the second engagement portion, and when the fixture is pulled upward, the case is removed from the hole.

8. The storage device according to claim 7, wherein
a movement direction for engaging with the case and a movement direction of the fixture for removing the case are opposite to each other.

9. The storage device according to claim 8, further comprising:
a cylindrical storage cabinet;
a disk pivotally supported to be rotatable about a center shaft of the storage cabinet provided inside the storage cabinet;
an opening through which the case and the fixture are capable of passing in an upper-lower direction in an upper surface of the storage cabinet; and
an opening and closing lid capable of opening and closing the opening, wherein
the holder has any one of a disk shape, a circular ring shape, and a fan shape rotating together with the disk,
the case is held by the holder concentrically with respect to the center shaft of the storage cabinet, and
when the opening and closing lid is opened, the fixture is lowered through the opening to engage with the case, the engagement between the case and the holder is released, and the case is taken out from the opening by raising the fixture.

10. An automatic analyzer comprising:
an analysis unit configured to measure a physical property of a sample; and
a storage device configured to store an article to be used for the measurement in the analysis unit, wherein
the storage device includes
a case in which the article is accommodated, and
a holder having a plurality of holes into each of which the case is capable of being inserted from above,
the holder includes a claw portion extending upward along a side surface of the case and elastically deformed in a direction away from a surface of the case, and
the case includes a first engagement portion that is engaged with the claw portion to fix the case when the case is inserted into the hole.

11. A storage method for an article comprising:
storing the article between a case in which the article, as a storage target, is accommodated and a holder having a plurality of holes into each of which the case is capable of being inserted from above, wherein
the holder includes a claw portion extending upward along a side surface of the case and elastically deformed in a direction away from a surface of the case, and
the case includes a first engagement portion that is engaged with the claw portion to fix the case when the case is inserted into the hole.
